# EUROPEAN PATENT SPECIFICATION

(11) **EP 2 853 282 B1**
(45) Date of publication and mention of the grant of the patent: **06.04.2016**
(21) Application number: 14173724.7
(22) Date of filing: 24.06.2014
(51) Int. Cl.: A61M 25/00, A61M 25/09

(54) **Guide wire**
Führungsdraht
Fil-guide

(30) Priority: 25.09.2013 JP 2013197685
(43) Date of publication of application: 01.04.2015
(73) Proprietor: ASAHI INTECC CO., LTD., Nagoya-shi, Aichi 463-0024 (JP)
(72) Inventor: Takada, Keigo, Nagoya-shi, Aichi 463-0024 (JP)
(74) Representative: Winter, Brandl, Fürniss, Hübner, Röss, Kaiser, Polte - Partnerschaft mbB

(56) References cited:
- EP-A1- 1 498 152
- EP-A1- 1 875 941
- EP-A2- 0 820 782
- EP-A2- 1 243 283
- WO-A1-97/48330

## Description

### [Technical Field]

The present invention relates to a guide wire inserted into the lumen of a blood vessel or the like.

### [Background Art]

Known is a guide wire used when inserting a catheter into a blood vessel. When inserting a catheter, first the guide wire is inserted into the blood vessel, and then the catheter is allowed to proceed along the guide wire. In such manner, the guide wire functions as a guide which guides the catheter to the lesion area.

As such a guide wire, commonly used is a so-called coil-type guide wire having the distal end portion of its core shaft covered with a coil body. In addition, for the purpose of ensuring lubricity within the blood vessel, proposed is a guide wire having the surface of its coil body covered with a coating film made of such as resin (Patent Literatures 1 to 4).

### [Citation List]

### [Patent Literature]

[Patent Literature 1] U.S. Patent No. 5840046
[Patent Literature 2] EP 1875941 A1
[Patent Literature 3] EP 0820782 A2
[Patent Literature 4] WO 97/48330 A1

### [Summary of Invention]

### [Technical Problem]

However, with the abovementioned conventional guide wire, by covering the surface of the coil body with a coating film, there was the problem of the coil body (and consequently the guide wire) becoming difficult to bend.

The present invention has been devised in light of the abovementioned problem exhibited by conventional technology and the object of the present invention is to provide technology that enables the coil body in a guide wire having the surface of its coil body covered with a coating film to be easily bendable.

### [Solution to Problem]

In order to solve the abovementioned problem, a guide wire according to the present invention according to claim 1 uses the following configuration. Namely, a guide wire comprising a core shaft, a coil body which covers said core shaft, and a coating film which covers said coil body, wherein said coating film is arranged to come between wires of the said coil body while gaps are formed between said wires and the portion of the coating film where said coating film comes between said wires, and the film thickness of the portions of the coating film that come between the wires of the coil body is less than the film thickness of the portions of the coating film that exist on the surface of the coil body.

In such a guidewire of the present invention, as the coating film is arranged to come between the wires of the coil body while gaps are formed between said wires and the portions where said coating film comes between said wires, it is possible for the coating film to easily bend when the coil body is bent. As a result, in the guide wire having the surface of its coil body covered with a coating film, the coil body (and consequently the guide wire) becomes easily bendable.

In such a guidewire of the present invention, as the film thickness of the coating film in portions that come between the wires of the coil body is less than the film thickness of the portions that exist on the surface of the coil body, it becomes easier for the coating film to bend when the guide wire is bent. As a result, as the coil body (and consequently the guide wire) can be made to be more easily bendable, it is possible for the guide wire to excellently follow an intricately tortuous blood vessel.

Furthermore, the portions that exist on the surface of the coil body are easily worn due to contact with exterior items (such as the inner walls of blood vessels or lesions). Accordingly, due to the film thickness of such portions being greater, it is also possible to apply resistance in relation to such wear.

In addition, in the abovementioned guide wire according to the present invention, the coating film which covers the coil body may be arranged such that the film comes between the wires to be deeper than the center of the wires of the coil body.

In such a guidewire of the present invention, as the coating film is arranged to come deeper than the center of the wires of the coil body, even in a case where the coil body is greatly bent (flexed) the coating film is prevented from becoming fully stretched. As a result, in addition to the abovementioned effect (of the coil body becoming easily bendable), even in a case where the guide wire is inserted into a bent region of a blood vessel, it is possible to prevent the coating film from rupturing due to being fully stretched.

In addition, although this will be described in detail later, due to the coating film being arranged to come deeper than the center of the wires of the coil body, it is also possible to improve the adhesion of the coating film and the coil body.

### [Brief Description of Drawings]

[Fig. 1] Fig. 1 is an explanatory diagram illustrating the configuration of a guide wire according to a first example (not part of the invention).
[Fig. 2] Fig. 2 is an enlarged view of the coil body and the coating film of the guide wire according to the first example (not part of the invention).
[Fig. 3] Fig. 3 is an enlarged view of the coil body and the coating film of a guide wire according to a second example (not part of the invention).
[Fig. 4] Fig. 4 is an enlarged view of the coil body and the coating film of a guide wire according to the embodiment of the present invention.

### [Description of Embodiments]

### A. First example (not part of the invention):

Various example of a guide wire are explained below in order to clarify the details of the abovementioned present invention.

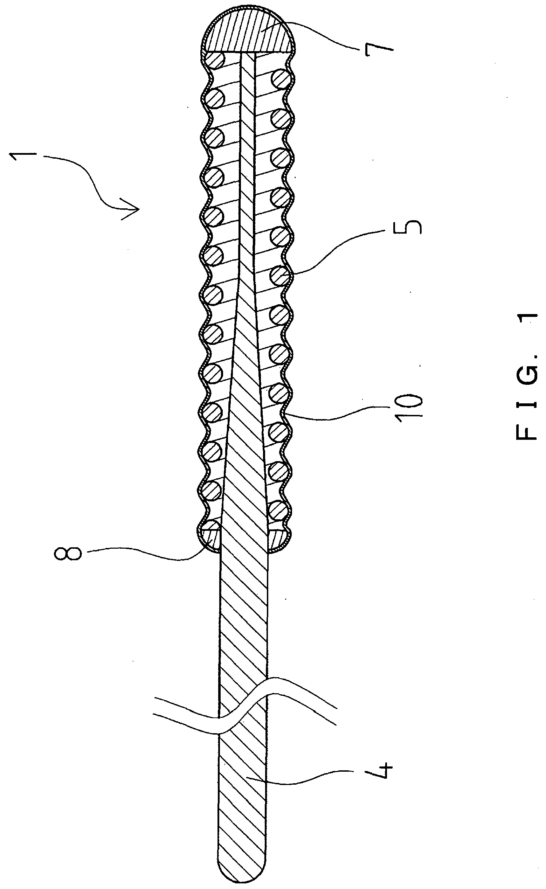

Fig. 1 is an explanatory diagram illustrating the configuration of guide wire (1) according to the first example. Guide wire (1) is configured of such as core shaft (4) and coil body (5) which covers core shaft (4). The distal end portion of coil body (5) and the distal end portion of core shaft (4) are connected via joint (7) while the base end portion of coil body (5) and the middle portion of core shaft (4) are connected via joint (8).

Furthermore, with guide wire (1) of this example, the surface of coil body (5) is covered with coating film (10). Coating film (10) is provided in order to ensure lubricity, when guide wire (1) is inserted into a blood vessel, by reducing the frictional resistance between the surface of guide wire (1) and the inner walls of the blood vessel. Therefore, it is preferable that coating portion (10) is formed of a material having low frictional resistance (such as a hydrophilic resin). For example, it is preferable that coating portion (10) is formed with polyvinyl alcohol, polyvinyl pyrrrolidone, polyethylene glycol, polyacrylamide, polyacrylic acid, sodium polyacrylate, poly-(2-hydroxyethyl methacrylate), a maleic acid copolymer, an ethylene vinyl alcohol copolymer, a monomer of 2-methacryloyloxyehtyl phosphorylcholine or a copolymer thereof, a (2-hydroxyethyl methacrylate)-styrene block copolymer, various synthetic polypeptides, collagen, hyaluronic acid, a cellulose-based polymer, or a mixture of any of these.

In addition, coating portion (10) may also be formed using a material where an additive such as a non-hydrophilic monomer, a cross-linking agent, a non-volatile solvent, a volatile solvent, or a surfactant is added to any of the abovementioned materials:

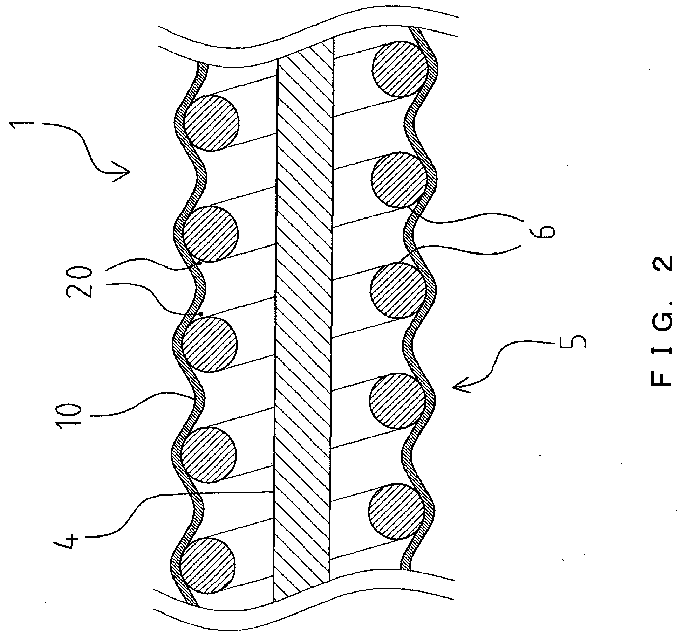

Fig. 2 is an enlarged view of coil body (5) and coating film (10) of guide wire (1) according to the first example. As illustrated in the drawing, coil body (5) of guide wire (1) of this example is formed such that adjacent wires (6) are not in contact with each other. In other words, the coil is formed in a state where the pitch of coil body (5) is spread out (open-coiled).

Furthermore, coating film (10) on the surface of coil body (5) is arranged to come between the wires of coil body (5). Due to this configuration, coating film (10) is formed to be in a so-called bellows-shape.

In this configuration, with guide wire (1) of this example gaps (20) are formed between the wires (6) of coil body (5) and the portions where the coating film (10) comes into the gaps between the wires of coil body (5). The reason for providing such gaps (20) in the guide wire (1) of this example is as explained below.

Namely, in a case where there is no gap between the wires of the coil body and the portions where the coating film come between the wires of the coil body (for example, in a case where the gaps are filled due to the film thickness of the coating film being rather thick), when the coil body is bent, tension is created on the coating film. As a result, since the coil body (and consequently the guide wire) is difficult to bend, this causes a negative effect where for example the ability of the guide wire to follow the shape of a blood vessel is lowered.

On the other hand, with guide wire (1) of this example since gaps (20) are formed between the wires (6) of coil body (5) and the portions where coating film (10) comes between the wires of coil body (5), it is easy for coating film (10) to bend when coil body (5) is bent. As a result, it becomes possible for coil body (5) to be easily bent

As explained in the above, with guide wire (1) of this example, even with a configuration having coating film (10) on the surface of coil body (5), coil body (5) can be easily bent. For this reason, it becomes possible to ensure lubricity of guide wire (1) within the blood vessel while also enabling the guide wire to follow the shape of the blood vessel in which it is inserted.

Along with the abovementioned first example, there exist other related example. The following are brief explanations of these other example. It shall be noted that in the following explanations, configurations that are identical to guide wire (1) of the first example are denoted by the same reference numerals and detailed explanations thereof are omitted.

### B. Second example (not part of the invention):

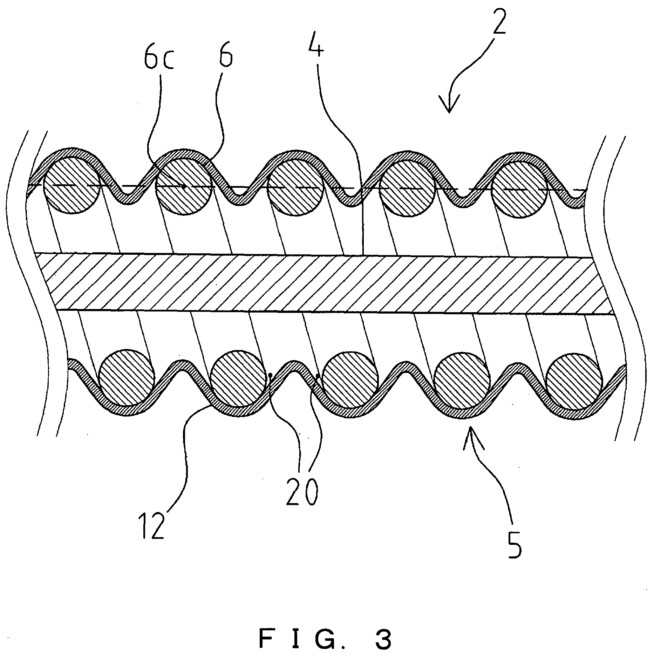

Fig. 3 is an enlarged view of coil body (5) and coating film (12) of guide wire (2) according to the second example. Guide wire (2) of is example differs from the abovementioned guide wire (1) of the first example in the following aspects. Namely, as illustrated in Fig. 3, coating film (12) which covers coil body (5) is, in relation to the gaps between the wires of coil body (5), arranged to come deeper than center (6c) of the wires (6). In other words, the depth of the drop of coating film (12) from the surface of coil body (5) is arranged to be greater than the radius of wire (6) of coil body (5).

Also In such a guidewire (2) of this example, similar to the abovementioned guide wire (1) of the first example, as gaps (20) are formed between the wires (6) of coil body (5) and the portions where coating film (12) come between the wires of coil body (5), it is possible for coil body (5) to be easily bent.

In addition, with guide wire (2) of this example, since coating film (12) is, in relation to the gaps between the wires of coil body (5), arranged to come deeper than center (6c) of the wires (6), even in a case where coil body (5) is greatly bent (flexed), coating film (12) is prevented from being fully stretched. Accordingly, even in a case where guide wire (2) is inserted into a bent region of a blood vessel, it is possible to prevent coating film (12) from rupturing due to being fully stretched.

Furthermore, as illustrated in Fig. 3, with guide wire (2) of this example, due to coating film (12) being, in relation to the gaps between the wires of coil body (5), arranged to come deeper than center (6c) of the wires (6), it is possible to ensure a sufficient contact area between coating film (12) and the wires (6) of coil body (5). As a result, it becomes possible to improve the adhesion of coating film (12) and coil body (5).

### C. Embodiment:

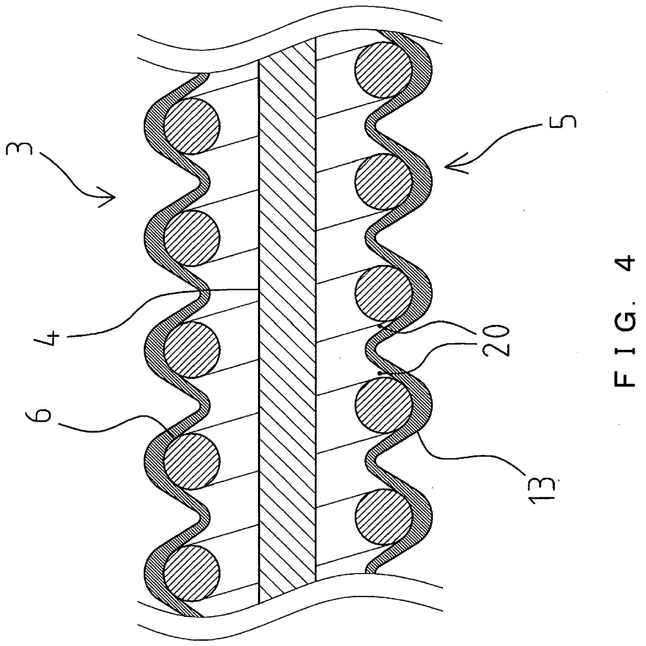

Fig. 4 is an enlarged view of coil body (5) and coating film (13) of guide wire (3) according to the embodiment. With the abovementioned guide wires of the first example and the second example, the film thickness of the coating films were explained as being uniform (refer to Fig. 2 and Fig. 3). Meanwhile, with guide wire (3) of this embodiment, the film thickness of coating film (13) differs depending on the portions. Namely, as illustrated in Fig. 4, with guide wire (3) of this embodiment, the film thickness of coating film (13) in portions where the film comes between the wires of coil body (5) is less than the film thickness of coating film (13) in portions where the film exists on the surface of coil body (5).

Also with such guide wire (3) of this embodiment, similar to the abovementioned guide wire (1) of the first example and guide wire (2) of the second example as gaps (20) are formed between the wires (6) and the portions where coating film (13) comes between the wires of coil body (5), it is possible for coil body (5) to be easily bent.

Furthermore, with guide wire (3) of this embodiment, it is possible for the guide wire to excellently follow an intricately tortuous blood vessel. This is due to the following reason.

Namely, when coil body (5) is bent, although the portions that exist on the surface of coil body (5) are barely subject to deformation, the portions that come between the wires of coil body (5) are subject to deformation. Accordingly, by reducing the film thickness of coating film (13) in such portions that are subject to deformation, it becomes easier for coating film (13) to bend when coil body (5) is bent. As a result, since it becomes easier to bend coil body (5) (and consequently guide wire (3)), it becomes possible for the guide wire to excellently follow an intricately tortuous blood vessel.

Meanwhile, the portions that exist on the surface of coil body (5) come into contact with exterior items (such as the inner walls of blood vessels or lesions) more frequently than the portions that come into the gaps between the wires of coil body (5) and accordingly are more easily worn. In this aspect, with guide wire (3) of this embodiment, since the film thickness is greater in the portions that are more subject to wear (portions that exist on the surface of coil body (5), it is also possible to improve the resistance of coating film (13) in relation to such wear.

With the abovementioned guide wires of the various example, the coating film was explained as not being configured to come further inward than the inner peripheral surface of the coil body (refer to Fig. 2 through Fig. 4). However, this coating film may be configured to be arranged in a further inward position than the inner peripheral surface of the coil body (drawing omitted).

However though, it is preferable that the coating film is arranged to not come too deep between the wires as this prevents the coating film from coming into contact with the core shaft when the guide wire is bent.

### [Reference Signs List]

- 1, 2, 3: Guide wire
- 4: Core shaft
- 5: Coil body
- 6: Wire
- 6c: Center of wire
- 7, 8: Joint
- 10, 12, 13: Coating film
- 20: Gap

## Claims

1. A guide wire (3) comprising:
a core shaft (4);
a coil body (5) which covers said core shaft (4); and
a coating film (10) which covers the coil body (5), wherein
the coating film (10) is arranged to come between wires (6) of the coil body while gaps (20) are formed between the wires (6) and the portions of the coating film (10) where the coating film (10) comes between said wires (6), **characterized in that** the film thickness of the portions of the coating film (10) that come between the wires (6) of the coil body (5) is less than the film thickness of the portions of the coating film (10) that exist on the surface of the coil body (5).

## Patentansprüche

1. Führungsdraht (3) mit:
einem Kernschaft (4);
einem den Kernschaft (4) umgebenden Wicklungskörper (5); und
einer den Wicklungskörper (5) umhüllenden Beschichtungsfolie (10), wobei
die Beschichtungsfolie (10) so angeordnet ist, dass sie zwischen die Wicklungsgänge (6) des Wicklungskörpers eindringt, wobei zwischen den Wicklungsgängen (6) und den Abschnitten der Beschichtungsfolie (10), wo die Beschichtungsfolie (10) zwischen die Wicklungsgänge (6) eindringt, Zwischenräume (20) vorgesehen sind, **dadurch gekennzeichnet, dass**
die Foliendicke der zwischen die Wicklungsgänge (6) des Wicklungskörpers (5) eingedrungenen Abschnitte der Beschichtungsfolie (10) kleiner ist als die Foliendicke der auf der Oberfläche des Wicklungskörpers (5) liegenden Abschnitte der Beschichtungsfolie (10).

## Revendications

1. Fil-guide (3) comprenant :
une tige centrale (4) ;
un corps hélicoïdal (5) qui recouvre ladite tige centrale (4) ; et
un film de revêtement (10) qui recouvre le corps hélicoïdal (5), dans lequel
le film de revêtement (10) est agencé pour venir entre des fils (6) du corps hélicoïdal alors que des espaces (20) sont formés entre les fils (6) et les parties du film de revêtement (10) où le film de revêtement (10) vient entre lesdits fils (6), **caractérisé en ce que** l'épaisseur de film des parties du film de revêtement (10) qui viennent entre les fils (6) du corps hélicoïdal (5) est plus petite que l'épaisseur de film des parties du film de revêtement (10) qui sortent sur la surface du corps hélicoïdal (5).
